# EUROPÄISCHE PATENTSCHRIFT

(11) **EP 3 616 211 B1**
(45) Veröffentlichungstag und Bekanntmachung des Hinweises auf die Patenterteilung: **13.09.2023**
(21) Anmeldenummer: 18720572.9
(22) Anmeldetag: 24.04.2018
(51) Int. Cl.: G16H 40/67, G16H 40/40

(54) **ÜBERWACHUNGSSYSTEM FÜR MINDESTENS EIN PERITONEAL-DIALYSEGERÄT**
MONITORING SYSTEM FOR AT LEAST ONE PERITONEAL DIALYSIS APPLIANCE
SYSTÈME DE SURVEILLANCE POUR AU MOINS UN APPAREIL DE DIALYSE PÉRITONÉALE

(30) Priorität: 24.04.2017 DE 102017206877; 14.02.2018 US 201815896695
(43) Veröffentlichungstag der Anmeldung: 04.03.2020
(73) Patentinhaber: Fresenius Medical Care Deutschland GmbH, 61352 Bad Homburg (DE)
(72) Erfinder: HOCHREIN, Torsten, 97478 Eschenau (DE); NADJA, Schubert, 97422 Schweinfurt (DE); FRANK, Hedmann, 97332 Volkach (DE)
(74) Vertreter: RCD Patent Giesen, Schmelcher & Griebel Patentwanwälte PartG mbB
(86) Internationale Anmeldenummer: PCT/EP2018/060423
(87) Internationale Veröffentlichungsnummer: WO 2018/197455

(56) Entgegenhaltungen:
- DE-A1-102014 017 897
- US-A- 5 629 871
- US-A1- 2014 230 071
- US-A1- 2016 206 800
- US-A9- 2004 027 244
- US-B1- 6 406 426

## Beschreibung

Die Erfindung betrifft ein Überwachungssystem für mindestens ein Peritoneal-Dialysegerät.

### Hintergrund

Eine Vielzahl von Menschen leidet unter Niereninsuffizienz oder einem chronischen oder akuten Nierenversagen. Zwar gibt es prinzipiell die Möglichkeit der Nierentransplantation, jedoch ist die Verfügbarkeit von passenden Spenderorganen begrenzt und eine Transplantation nicht bei jedem Patienten durchführbar. Daher wurden in der Vergangenheit Verfahren zur Blutwäsche, der sogenannten Dialyse, entwickelt. Dialyse als Vorgang ist dadurch gekennzeichnet, dass mittels einer Membran ein Stoffaustausch vorgenommen wird. Dabei befindet sich auf einer Seite der Membran die zu reinigende Flüssigkeit und auf der anderen Seite eine geeignete Dialyselösung.

Dabei wurden in der Vergangenheit verschiedene Verfahren etabliert. So gibt es die Hämodialyse, bei der mittels einer semipermeablen Membran Blut von Harnstoff, Harnsäure und anderen Stoffen aus dem Blut unter Einfluss von osmotischem Druck aus dem Blut herausgelöst wird. Andere Verfahren sind die Hämoperfusion, Hämodiafiltration und die Hämofiltration.

Weiterhin wurde in der Vergangenheit auch das Verfahren der Peritonealdialyse entwickelt. Die Peritonealdialyse (PD) ist auch unter dem Begriff Bauchfelldialyse bekannt. Dabei werden unter dem Begriff auch unterschiedliche Verfahren, wie z.B. das manuelle CAPD (continuously ambulatory peritoneal dialysis, kontinuierliche ambulante Peritonealdialyse), oder die mit Geräten durchgeführten Verfahren der CCPD (kontinuierliche zyklische PD), der IPD (intermittierende PD), NIPD (nächtliche intermittierende PD) etc. unter dem Begriff der Peritonealdialyse zusammengefasst.

Diese Verfahren basieren darauf, dass das Bauchfell (Peritoneum) eine Haut ist, die die Bauchhöhle auskleidet und gut durchblutet ist.

Aufgrund des besonderen Aufbaus kann das Peritoneum ebenfalls als "Filtermembran" genutzt werden. Patienten wird hierzu in aller Regel ein Schlauch (Katheter) in die Bauchhöhle implantiert. Über diesen Katheter wird eine Dialyselösung in den Bauchraum gefüllt und dort über einen gewissen Zeitraum belassen. Kleinmolekulare Substanzen können nun aus dem Blut über die Kapillargefäße des Bauchfells in die Dialyselösung übertreten, da ein Konzentrationsgefälle herrscht. Die Dialyselösung muss nach einer bestimmten Zeit abgelassen und durch eine frische ersetzt werden.

Im Unterschied zur künstlichen Membran bei der Blutdialyse ist das Peritoneum auch für Eiweiße recht durchlässig, wodurch es zu einem relevanten Eiweißverlust kommt.

Die Peritonealdialyse erweist sich aber in vielerlei Hinsicht als vorteilhaft. Die Peritonealdialyse erlaubt im Unterschied zu anderen Dialyseverfahren eine längere Aufrechterhaltung einer eventuell noch vorhandenen Restnierenfunktion. Zudem werden bei der Peritonealdialyse seltener Komplikationen berichtet, da der Kreislauf weniger stark belastet wird. Dies ist insbesondere bei Patienten mit Herzproblemen ein großer Vorteil. Grundsätzlich kann eine Peritonealdialyse auch durch Patienten im häuslichen Bereich angewendet werden während eine Hämodialyse nur in speziell eingerichteten Zentren/Praxen/Kliniken zur Verfügung steht. Trotz dieser Vorteile ist der Verbreitungsgrad der Peritonealdialyse gering. Dies ist unter anderem darin begründet, dass die Sicherstellung des einwandfreien Betriebes der entsprechenden Geräte nicht zuverlässig bereitgestellt werden kann. Dies ist unter anderem darin begründet, dass die Peritoneal-Dialysegeräte auch einem Verschleiß unterliegen, der unter anderem abhängig ist vom Gebrauch.

Aus der US-Patentanmeldung US 2016 / 206 800 A1 ist ein entferntes Überwachungsschnittstellengerät und eine mobile Anwendung für ein medizinisches Gerät bekannt, dass Behandlungsdaten streamt. Aus der US Patentanmeldung US 2014 / 230 071 A1 ist ein Gerät und ein Verfahren zur Erstellung und Bereitstellung eines grafischen Codes bekannt, der spezifisch ist für ein medizinisches Gerät und eine medizinische Behandlung. Aus der US5629871 ist die Auswertung der von Dialysegeräten empfangenen Daten hinsichtlich Verschleiß bekannt. Die zuvor genannten Schriften zeigen jedoch nicht den Erhalt von Fahrwegprofildaten noch deren Auswertung hinsichtlich Verschleiß.

### Aufgabe

Es ist daher eine Aufgabe der Erfindung ein Überwachungssystem für Peritoneal-Dialysegeräte bereitzustellen, mit dem eine erhöhte Sicherheit für den Patienten zur Verfügung gestellt werden kann.

### Kurzdarstellung der Erfindung

Die Aufgabe wird gelöst durch ein Überwachungssystem für mindestens ein Peritoneal-Dialysegerät, wobei das Überwachungssystem von einem zu überwachendem Peritoneal-Dialysegerät mittels eines ersten Datenübertragungsnetzes Fahrwegprofildaten erhält, wobei die erhaltenen Daten bezogen auf ein jeweiliges Peritoneal-Dialysegerät gespeichert werden, wobei gerätebezogen zumindest einzelne der gespeicherten Daten ausgewertet werden, wobei auf Basis der Auswertung eine Aktion ausgewählt aus Benachrichtigen eines Patienten, Benachrichtigen eines medizinischen Fachpersonals, Benachrichtigen von Servicepersonal, Benachrichtigen eines Qualitätsbeauftragten mittels eines zweiten Datenübertragungsnetzes ausgeführt wird, wobei das zu überwachende Peritoneal-Dialysegerät (PD1) eine Membranpumpe aufweist, wobei mittels Auswertung von Änderungen eines Fahrwegprofils Verschleißdaten ermittelt werden.

Weitere vorteilhafte Ausgestaltungen sind Gegenstand der abhängigen Ansprüche, der Figuren und der ausführlichen Beschreibung.

### Kurzdarstellung der Figuren

Nachfolgend wird die Erfindung näher unter Bezugnahme auf die Figuren erläutert werden. In diesen zeigt:
Fig. 1 eine schematische Übersicht auf ein Überwachungssystem und Peritoneal-Dialysegeräte gemäß Ausführungsformen der Erfindung,
Fig. 2 eine schematische Übersicht von Komponenten eines Peritoneal-Dialysegerätes gemäß Ausführungsformen der Erfindung, und
Fig. 3 eine schematische Übersicht von Komponenten eines Überwachungssystems gemäß Ausführungsformen der Erfindung.

### Detaillierte Beschreibung

Nachfolgend wird die Erfindung eingehender unter Bezugnahme auf die Figur dargestellt werden. Dabei ist anzumerken, dass unterschiedliche Aspekte beschreiben werden, die jeweils einzeln oder in Kombination zum Einsatz kommen können. D.h. jeglicher Aspekt kann mit unterschiedlichen Ausführungsformen der Erfindung verwendet werden, soweit nicht explizit als reine Alternative dargestellt.

Weiterhin wird nachfolgend der Einfachheit halber in aller Regel immer nur auf eine Entität Bezug genommen werden. Soweit nicht explizit vermerkt, kann die Erfindung aber auch jeweils mehrere der betroffenen Entitäten aufweisen. Insofern ist die Verwendung der Wörter "ein", "eine" und "eines" nur als Hinweis darauf zu verstehen, dass in einer einfachen Ausführungsform zumindest eine Entität verwendet wird.

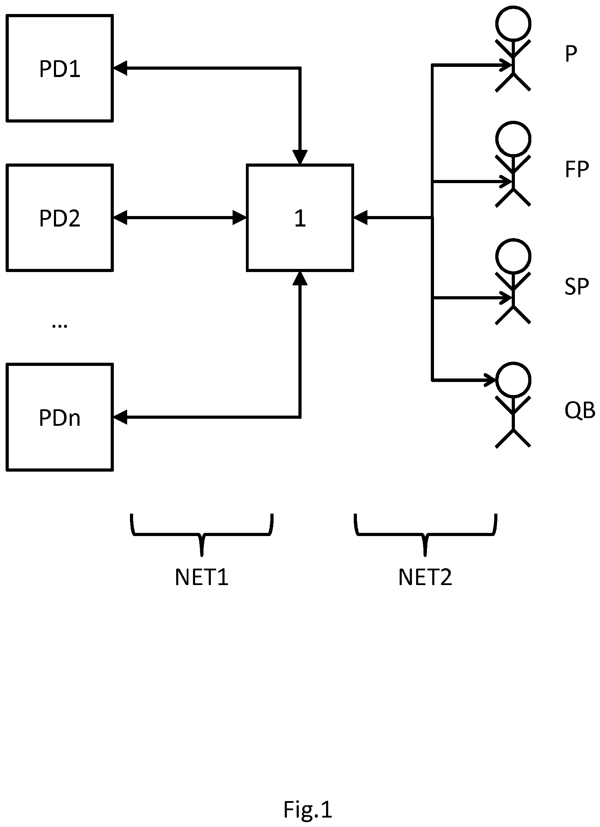

Ein erfindungsgemäßes Überwachungssystem 1 ist in seinem Kontext in Figur 1 dargestellt. Das Überwachungssystem 1 ist zur Überwachung von mindestens einem Peritoneal-Dialysegerät PD1 in aller Regel jedoch einer Vielzahl von Peritoneal-Dialysegeräten PD1 - PDn geeignet.

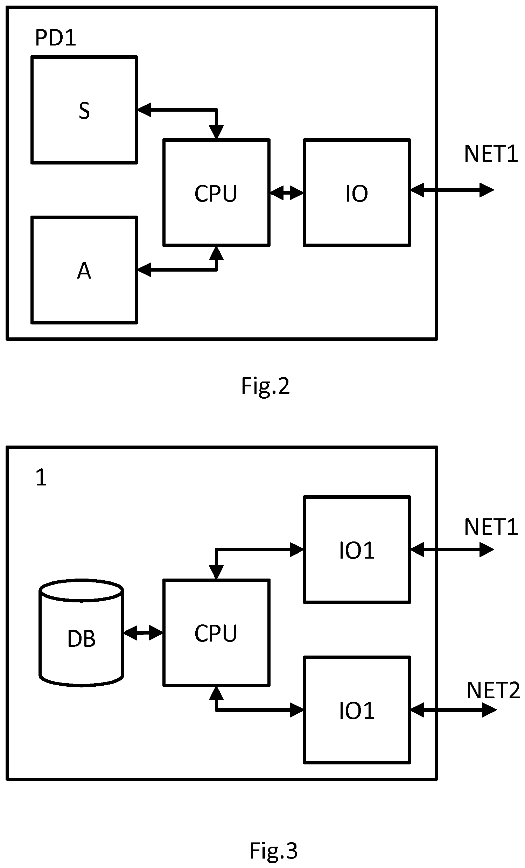

Ein beispielhaftes Peritoneal-Dialysegerät PD1 ist in Figur 2 dargestellt. Das Peritoneal-Dialysegerät PD1 weist z.B. einen oder mehrere Sensoren S, einen oder mehrere Aktoren A, zumindest eine CPU zur Verarbeitung von Sensordaten und/oder zur Ansteuerung der Aktoren A, sowie mindestens eine Kommunikationseinheit IO, die zur Übertragung an das jeweilige erste Datenübertragungsnetz NET1 ausgestaltet ist, auf.

Das Überwachungssystem 1 erhält von mindestens dem einem zu überwachendem Peritoneal-Dialysegerät PD1 oder von einer Auswahl oder von allen Peritoneal-Dialysegeräten PD1 - PDn mittels eines ersten Datenübertragungsnetzes NET1 Daten.

Das Datenübertragungsnetz NET1 kann jegliches geeignete drahtlose oder drahtgebundene Datenübertragungsnetz sein. Zudem ist die Wahl des Übertragungsprotokolles der jeweiligen Implementierung überlassen. Zudem ist der Begriff eines Datenübertragungsnetzes nicht limitierend aufzufassen, sondern es können unterschiedliche Datenübertragungsnetze für unterschiedliche Daten oder auch redundante Datenübertragungsnetze vorgesehen sein. Zudem kann der Mechanismus zur Bereitstellung von Daten die aktive Übersendung durch einen Benutzereingriff am Peritoneal-Dialysegerät PD1, und/oder die periodische oder eventbezogene Versendung von Daten durch das Peritoneal-Dialysegerät PD1 und/oder die Abfrage von Daten durch das Überwachungssystem 1 (periodisch, aperiodisch, auf Anfrage) beinhalten.

Die Daten, welche vom Peritoneal-Dialysegerät PD1 bereitgestellt oder von diesem durch das Überwachungssystem 1 abgefragt werden, sind ausgewählt aus einer Gruppe aufweisend Gerätedaten, Fehlercodes, Betriebsdaten, Umweltdaten, Verbrauchsmitteldaten, Netzwerkdaten, Behandlungsverlaufdaten.

Ein beispielhaftes Überwachungssystem 1 ist in Figur 3 dargestellt. Das Überwachungssystem 1 weist z.B. zumindest eine CPU zur Verarbeitung von Daten und/ ein Datenbank DB oder einen anderen lokalen oder entfernten Datenspeicher, sowie mindestens eine erste logische Kommunikationseinheit IO1, die zur Übertragung an das jeweilige erste Datenübertragungsnetz NET1 ausgestaltet ist, und eine zweite logische Kommunikationseinheit IO2, die zur Übertragung an das jeweilige zweite Datenübertragungsnetz NET2 ausgestaltet ist, auf.

Es sei angemerkt, dass das erste Datenübertragungsnetz NET1 und das zweite Datenübertragungsnetz NET2 als logische Datenübertragungsnetze getrennt dargestellt sind. Es ist jedoch keine zwingende Voraussetzung, dass die Datenübertragungsnetze unterschiedlich sind. Vielmehr kann das erste Datenübertragungsnetz NET1 und das zweite Datenübertragungsnetz NET2 Bestandteil eines Datennetzes sein.

Im Überwachungssystem 1 werden die vom dem Peritoneal-Dialysegerät PD1 erhaltenen Daten bezogen auf ein jeweiliges Peritoneal-Dialysegerät PD1 z.B. in einer lokalen oder entfernten Datenbank DB oder einem lokalen oder entfernten Datenspeicher gespeichert.

Im Überwachungssystem 1 können sodann gerätebezogen zumindest einzelne der gespeicherten Daten ausgewertet werden, wobei auf Basis der Auswertung eine Aktion ausgewählt aus Benachrichtigen eines Patienten P, Benachrichtigen eines medizinischen Fachpersonals FP, Benachrichtigen von Servicepersonal (SP), Benachrichtigen eines Qualitätsbeauftragten QB mittels eines zweiten Datenübertragungsnetzes NET2 ausgeführt wird.

Beispielsweise könnte dies so implementiert sein, dass die Peritoneal-Dialysemaschine PD1 mittels Email/SMS oder ähnlichen Diensten maschinen- und maintenance-bezogenen Daten an das Überwachungssystem 1 bereitstellt. Das Überwachungssystem 1 extrahiert Zahlenwerte aus den übertragenen Daten. Im Überwachungssystem 1 erfolgt eine Auswertung im Hinblick auf Fehlerfall, Verschleiß, sowie ein Einordnen der Ergebnisse im Hinblick auf Schwere des Fehlers und Grad des Verschleißes.

Beispielsweise wird den Fehlern ein Fehlercode zugewiesen.

In Abhängigkeit von dem Fehlercode erfolgt ein Routing zu einer entsprechenden Nutzergruppe. D.h. für jeden Fehlercode gibt es eine klare Zuweisung an eine oder mehrere Nutzergruppen.

Z.B. bei einem Fehlerfall, der einen Einsatz eines Servicetechnikers erforderlich macht, wird der erkannte Fehler und die Angabe der fehlerhaften Peritoneal-Dialysemaschine PD1 an das Service Personal SP weitergeleitet.

Ein Fehlerfall, der auf eine mangelnde Schulung des Bedieners schließen lässt, wird z.B. an einen Qualitätsbeauftragten QB geleitet. Dies kann z.B. ein besonderer Anwendungsberater oder auch medizinisches Fachpersonal, wie z.B. Ärzte oder Krankenschwestern sein.

Beispielsweise können die Daten von der jeweiligen Peritoneal-Dialysemaschine PD1 an eine zentrale Adresse / Postfach versandt werden, die dem Überwachungssystem 1 zugeordnet ist. Das Überwachungssystem 1 sortiert dann die erhaltenen Daten z.B. nach Absender (entsprechend der Peritoneal-Dialysemaschine) ein. So sind die entsprechenden Daten auf dem Überwachungssystem 1 in Bezug auf ein bestimmtes Gerät abgespeichert.

Auf dem Überwachungssystem 1 kann z.B. ein WWW-Server vorgesehen sein. Dieser kann zum einen die Daten gerätespezifisch aufbereiten und (farblich hervorgehoben und/oder sortiert) darstellen, zum anderen auch auf Anforderung bestimmte Analysen in den Daten (gerätebezogen) durchführen.

D.h. neben der aktiven Benachrichtigung wird auch die Möglichkeit bereitgestellt, dass bestimmte Nutzergruppen (gerätebezogene) Analysen in den Daten anstoßen und die Ergebnisse abfragen können. Die Ergebnisse können dabei auf dem Webserver bereitgestellt werden oder aber mittels Email/SMS an eine für den jeweiligen Nutzer hinterlegten Adresse an den jeweiligen Empfänger, z.B. den Patient P, einen Arzt und/oder Pflegepersonal FP , einen Servicepersonal SP, einen Qualitätsbeauftragten QB, und weitere geleitet werden.

Es sei angemerkt, dass die Analyse und Benachrichtigungsfunktionen konfigurierbar gestaltet sein können.

Beispielsweise können schwerwiegende Fehler einen unmittelbaren Einsatz von Servicepersonal SP auslösen. Andere Fehler, die auf Verschleißerscheinung hindeuten, können zusammen mit entsprechenden Daten und/oder Auswertungen an das Servicepersonal SP weiterlgeleitet werden. Beispielsweise kann anhand des Druckverlaufs einer Membranpumpe (aufgenommen durch einen Sensor S) bestimmt werden, ob der Überdruck- bzw. Unterdruckaufbau in entsprechenden Intervallen bzw. Geschwindigkeiten geschieht. Weiterhin kann z. B. aus dem Druckverlauf auch erkannt werden, ob der Druck stabil ist. Aus anderen und/oder zusätzlichen Daten kann erkannt werden, ob die Ventile zuverlässig schließen / öffnen. Zudem kann z.B. ein Initalisierungstest anzeigen, dass bestimmte Elemente der Peritoneal-Dialysemaschine an ihre Grenzen stoßen. Auch kann z.B. die Überwachung von Sensornullpunkten, Linearität von Längensensoren etc. erkannt werden. Aus der Anzahl von Entgasungszyklen kann ein Rückschluss auf die Dichtigkeit von Hydraulikelementen gezogen werden. Zudem kann z.B. allgemein der aufgenommene Strom überwacht werden. Ist dieser aufgenommene Strom vergleichsweise hoch, so deutet dies auf eine erhöhte innere Reibung, z.B. Verlust eines Schmiermittels, Abrieb, etc. hin. Zudem kann die generelle Geschwindigkeit von einzelnen Komponenten erfasst werden. Auch hieraus lassen sich Rückschlüsse auf innere Reibung ziehen. Zudem kann auch die Heizleistung erfasst werden. Steigt diese an, so ist dies z.B. ein Hinweis auf einen Fehler im Heizsystem. Andere Daten, wie z.B. fehlerhafte Erkennung von Barcodes innerhalb kurzer Frist lassen auf einen Fehler der optischen Eigenschaften eines Barcodeleser-Subsystems der Peritoneal-Dialysemaschine schließen. Andere Parameter, wie z.B. in der Peritoneal-Dialysemaschine eingesetzte Datenspeicher oder elektrische Akkus können ebenfalls Zustandsdaten zur Verfügung stellen.

D.h. mittels des Überwachungssystems 1 werden mittels Auswertung aus den Daten Verschleißdaten ermittelt. Insbesondere werden bei einem zu überwachenden Peritoneal-Dialysegerät PD1, das eine Membranpumpe aufweist, mittels Auswertung von Änderungen eines Fahrwegprofils Verschleißdaten ermittelt. Weiterhin ist es möglich mittels Auswertung von Änderungen von Druck- und/oder Fahrwegprofil(en), d.h. Verlaufsdaten, Verschleißdaten zu ermitteln.

In einer Ausführungsform erfolgt eine Feststellung des Verschleißes der Pumpeneinheit anhand eines Druck-/Fahrwegprofils einer Membranpumpe in der PD-Maschine, ein Feststellen des Verschleißgrades erfolgt in Abhängigkeit von Änderungen des festgestellten Druck-/Fahrwegprofils.

Obwohl in der bisherigen Beschreibung die Überwachung der Peritoneal-Dialysemaschine an sich im Vordergrund gestanden hat, erlaubt das Überwachungssystem 1 auch die Erkennung von Chargenstreuung bei verwendeten Einwegartikel. So kann z.B. aus der Bewertung von Luftdetektionen ein Hinweis auf Undichtigkeiten an den unterschiedlichen Verbindern zum Patienten P gezogen werden.

Ohne Weiteres können in der Peritoneal-Dialysemaschine auch andere Sensoren vorhanden sein, um z.B. unzulässige Umgebungsbedingungen zu erkennen. So kann z.B. die Umgebungstemperatur ermittelt werden, um hieraus z.B. zu bestimmen, ob die Heizleistung überhaupt ausreichen kann. Zudem kann z.B. der Luftdruck ermittelt werden, um z.B. die Leistungsfähigkeit eines Kompressors beurteilen zu können. Auch die Luftfeuchtigkeit kann ermittelt werden, um z.B. Schädigungen der Elektronik, Korrosion durch kondensierende Luftfeuchtigkeit frühzeitig erkennen zu können. Zudem ist es möglich, das elektrische Netz zu überwachen, das die Peritoneal-Dialysemaschine mit Energie versorgt.

Andere Fehler deuten hingegen auf eine fehlerhafte Schulung im Umgang mit der Peritoneal-Dialysemaschine hin. Mögliche Katheter-Probleme können über die Anzahl der Ausführung der "Wake Up" Funktionalität und/oder über die Bestimmung des Anstieges eines Volumen-Zeit Diagrammes ermittelt werden. Auch können etwaige Kürzungen der verschriebenen Einlaufvolumen ermittelt werden, die einen Hinweis auf mangelnde Compliance sind. Zudem können Umschaltpunkte zwischen Ein- und Auslauf erfasst und über eine Vielzahl von Einläufen bewertet werden. Auch dies kann einen Hinweis auf mangelnde Compliance sein. Zudem kann aus der generellen Überwachung, ob überhaupt eine Behandlung erfolgt, überwacht werden. Aus der Anzahl der Anzeige von Fehlermeldungen kann auf eine mangelnde Schulung im Umgang mit der Peritoneal-Dialysemaschine geschlossen werden. Auch die Zeiträume zwischen einer Bereitstellung einer Behandlung und dem tatsächlichen Start der Behandlung kann ein Hinweis auf eine mangelnde Schulung sein, denn ein zu langer Abstand kann eine Verkeimung nach sich ziehen. In diesem Fall kann z.B. ein Qualitätsbeauftragter QB informiert werden, der eine erneute Schulung des Patienten P veranlasst.

Die entsprechenden Auslösemechanismen können auf dem Überwachungssystem 1 konfiguriert werden. Beispielsweise können die Auslöseparameter spezifiziert werden. Zudem ist es möglich die zu kontaktierenden Personen oder den Kontaktweg zu konfigurieren. Dabei können bestimmte Personen und/oder Geräte auch gruppiert werden, sodass z.B. für bestimmte Patienten oder für bestimmte Geräte immer gleiche Parameter gehören.

Ohne Weiteres kann aber auch das Profil der zu übertragenden Daten vom Überwachungssystem 1 auf den entsprechenden Peritoneal-Dialysemaschinen konfiguriert werden.

Aus den Gerätedaten erstellt der Server eine gerätespezifische Auswertung, welche die technischen Gerätedaten und die Therapiedaten extrahiert und visualisiert.
∘ Betriebsstunden
∘ Dauerlaufdaten
∘ Gerätefehler mit Fehlerstatistik
∘ Verfahrensparameter wie z.B. Pumpenparameter, Messergebnisse von Initialtests, Sensorwerte, ...
∘ Angezeigter Bildschirmverlauf
∘ Gesamtes Einlauf- Auslauf Volumen
∘ Gesamte Verweilzeiten
∘ Einlaufreduzierung
∘ Verweilzeitreduzierung
∘ UF

Die Analyseregeln sind auf dem Server integriert und erlauben es dem Server, die Gerätedaten auf bestimmte Ereignisse bzw. Muster zu untersuchen.

Es können z.B. die folgenden Daten genutzt werden.
∘ Umgebungsbedingungen
∘ Reparaturzustand {Verschleißdaten, Betriebsstunden, Technikereinsatz, erneuerte Komponenten, Ausstattungscode, Gerätefehler}

Aus dieser Analyse können verschiedene Schlussfolgerungen für das Gerät ermittelt werden.
- Reparaturempfehlung für Service Techniker
- Anpassung des Aufzeichnungsverhaltens der Geräte
- Vertiefte Rückschlüsse auf Chargenstreuung der Einwegartikel
- Trenderkennung von Komponentenausfall der Geräte.
- Anpassung der Behandlung bzw. Patientenbetreuung
- Nutzungsverhalten des Gerätes

In der Datenbank DB bzw. dem Speicher werden die erhaltenen Daten bevorzugt verknüpft mit einem Zeitpunkt abgespeichert.

Treten Fehler in einem vorbestimmten Zeitraum häufiger auf, so können durch das Überwachungssystem 1 zusätzliche Daten von der jeweiligen Peritoneal-Dialysemaschine angefordert werden, die eine erweiterte Analyse ermöglichen.

Aus der Häufigkeit innerhalb einer bestimmten Zeit (Fehlerrate) kann z.B. abgeschätzt werden, ob ein Fehler akut ist bzw. wie dringend eine Fehlerbehebung am Gerät notwendig ist.

Treten Fehler in einem vorbestimmten Zeitraum seltener auf, so können durch das Überwachungssystem 1 weniger Daten von der jeweiligen Peritoneal-Dialysemaschine angefordert werden, die eine weniger umfangreiche Analyse ermöglichen.

Das Überwachungssystem 1 filtert aus von der Peritoneal-Dialysemaschine erhaltenen Daten einzelne oder mehrere spezifische Maschinendaten und Dauerlaufdaten heraus und speichert diese ab.

Zu den spezifischen Maschinendaten gehören eine eindeutige Gerätenummer, eine eindeutige Nummer für die Hardwareausstattung der Peritoneal-Dialysemaschine, eine eindeutige Kennzeichnung der aktuell installierten Softwareversion, das Land in welches die Peritoneal-Dialysemaschine ausgeliefert wurde, die Sprache mit welcher die Peritoneal-Dialysemaschine ausgeliefert wurde sowie das Produktionsdatum und das Datum des letzten Einsatzes eines Service-Technikers an der Peritoneal-Dialysemaschine.

Die Dauerlaufdaten beinhalten die Betriebsstunden der Peritoneal-Dialysemaschine seit Auslieferung, die Betriebsstunden der Peritoneal-Dialysemaschine seit dem letzten Service-Einsatz sowie die Anzahl der Schaltvorgänge von Ventilen, Motoren und Heizungen seit Auslieferung der Peritoneal-Dialysemaschine.

Zusätzlich können aus allen für das Überwachungssystem 1 verfügbaren Daten die Anzahl der Behandlungen bestimmt werden

Die spezifischen Maschinendaten werden extrahiert um u.a. zu analysieren, ob bestimmte Fehlerschemata in Verbindung mit der eingesetzten Software oder mit dem Hardwarestand der Peritoneal-Dialysemaschine stehen. Umgekehrt ist es möglich, z.B. bei Bekanntwerden von Produktionsfehlern alle betreffenden Peritoneal-Dialysemaschinen einer bestimmten Softwareversion oder eines bestimmten Hardwarestandes zu identifizieren und eine automatische Nachricht an den Techniker zur Wartung dieser Peritoneal-Dialysemaschine zu schicken.

Die Anzahl der Betriebsstunden in Verbindung mit den Schaltvorgängen elektromechanischer Komponenten sowie bestimmten Fehlerschemata werden analysiert um den Verschleiß mechanischer/elektrischer Bauteile zu überwachen und ggf. das Service-Personal SP über den bevorstehenden Austausch dieser Teile zu informieren. Weiterhin wird anhand der Betriebsstunden eine Erinnerung über den nächsten Wartungszeitpunkt an das Service-Personal versendet.

Eine langfristige Überwachung der Dauerlaufdaten ermöglicht es u.a. das Verhalten, den Verschleiß und die Robustheit von Hydraulik-und Pneumatik-Systemen der Peritoneal-Dialysemaschine zu analysieren und zu bewerten.

So können frühzeitig Produktverbesserungen angestoßen werden.

Die Daten zu Aufstellland und Sprache der Peritoneal-Dialysemaschine werden u.a. extrahiert um einen Rückschluss auf länderspezifische Probleme z.B. durch extreme Klimabedingungen (Temperatur, Luftfeuchte) oder spezielle Handhabung zu ziehen.

Soweit vorstehend ein drahtloses oder drahtgebundenes Datenübertragungsnetz NET1 bzw. NET2 angesprochen wird, ist für den Fachmann ersichtlich, dass hier insbesondere ein lokales Netzwerk bis hin zum globalen Netzwerk Internet gemeint sein kann, wobei jegliches geeignetes Medium, wie z.B. Draht, Glasfaser oder Mobilkommunikation (Bluetooth, ZigBee, WLAN, GSM,UMTS,LTE,LTE-A, und nachfolgende Technologien) umfasst sind.

Eine Trennung in zwei (virtuelle) Netze wird als vorteilhaft angesehen, da hiermit der direkte Zugriff auf Peritoneal-Dialysemaschine als auch auf Daten der Peritoneal-Dialysemaschine, die in dem Überwachungssystem 1 gespeichert sind, vermieden werden kann. Hierdurch können unberechtigte Zugriffe verhindert werden.

Ohne Weiteres kann in einer Ausführungsform der Erfindung vorgesehen sein, dass eine bestimmte Auswertung durch einen Benutzer P, SP, QM, FP angefordert werden kann.

Wie bereits zuvor beschrieben sind zumindest einzelne der gespeicherten Daten und Auswertungen der betreffenden gespeicherten Daten mittels eines Web-Browsers abrufbar.

Insbesondere wenn unverschlüsselte Kommunikationsmittel verwendet werden ist es vorteilhaft, wenn die Daten des Peritoneal-Dialysegerät PD1 verschlüsselt übertragen werden.

In einer Ausführungsform der Erfindung werden die Benachrichtigungen mittels eines Messaging-Dienstes versandt. D.h. das System kann in bestehende Kommunikationsinfrastrukturen eingepasst werden.

Obwohl vorstehend die Erfindung in Bezug auf ein Peritoneal-Dialysegerät PD1 dargestellt wurde, ist die Erfindung nicht hierauf beschränkt. Vielmehr kann das Überwachungssystem 1 eine Vielzahl von Peritoneal-Dialysegeräten (PD1, PD2, ... PDN) - wie in Figur 1 gezeigt - überwachen.

## Patentansprüche

1. Überwachungssystem (1) für mindestens ein Peritoneal-Dialysegerät (PD1), wobei das Überwachungssystem (1) von einem zu überwachendem Peritoneal-Dialysegerät (PD1) mittels eines ersten Datenübertragungsnetzes (NET1) Fahrwegprofildaten erhält, wobei die erhaltenen Daten bezogen auf ein jeweiliges Peritoneal-Dialysegerät (PD1) gespeichert werden, wobei gerätebezogen zumindest einzelne der gespeicherten Daten ausgewertet werden, wobei auf Basis der Auswertung eine Aktion ausgewählt aus Benachrichtigen eines Patienten (P), Benachrichtigen eines medizinischen Fachpersonals (FP), Benachrichtigen von Servicepersonal (SP), Benachrichtigen eines Qualitätsbeauftragten (QB) mittels eines zweiten Datenübertragungsnetzes (NET2) ausgeführt wird, wobei das zu überwachende Peritoneal-Dialysegerät (PD1) eine Membranpumpe aufweist, wobei mittels Auswertung von Änderungen des Fahrwegprofils Verschleißdaten der Membranpumpe ermittelt werden.

2. Überwachungssystem (1) nach Anspruch 1, **dadurch gekennzeichnet, dass** das erste Datenübertragungsnetz (NET1) ein drahtloses oder drahtgebundenes Datenübertragungsnetz ist.

3. Überwachungssystem (1) nach Anspruch 1 oder 2, **dadurch gekennzeichnet, dass** das zweite Datenübertragungsnetz (NET2) ein drahtloses oder drahtgebundenes Datenübertragungsnetz ist.

4. Überwachungssystem (1) nach einem der vorhergehenden Ansprüche, **dadurch gekennzeichnet, dass** die Auswertung durch einen Benutzer angefordert werden kann.

5. Überwachungssystem (1) nach einem der vorhergehenden Ansprüche, **dadurch gekennzeichnet, dass** zumindest einzelne der gespeicherten Daten und Auswertungen der betreffenden gespeicherten Daten mittels eines Web-Browsers abrufbar sind.

6. Überwachungssystem (1) nach einem der vorhergehenden Ansprüche, **dadurch gekennzeichnet, dass** die Daten des Peritoneal-Dialysegerät (PD1) verschlüsselt übertragen werden.

7. Überwachungssystem (1) nach einem der vorhergehenden Ansprüche, **dadurch gekennzeichnet, dass** die Benachrichtigungen mittels eines Messaging-Dienstes versandt werden.

8. Überwachungssystem (1) nach einem der vorhergehenden Ansprüche, wobei mittels Auswertung von Änderungen von Druckprofil(en) Verschleißdaten ermittelt werden.

9. Überwachungssystem (1) nach einem der vorhergehenden Ansprüche, **dadurch gekennzeichnet, dass** das Überwachungssystem (1) eine Vielzahl von Peritoneal-Dialysegeräten (PD1, PD2, ... PDN) überwacht.

10. Überwachungssystem (1) nach einem der vorhergehenden Ansprüche, **dadurch gekennzeichnet, dass** das Aufzeichnungsverhalten eines Peritoneal-Dialysegerätes (PD1) aus der Vielzahl von Peritoneal-Dialysegeräten (PD1, PD2, ... PDN) als Antwort auf einen Vergleich der aktuellen Fehlerrate mit einer Bezugsfehlerrate angepasst wird

11. Überwachungssystem (1) nach einem der vorhergehenden Ansprüche, **dadurch gekennzeichnet, dass** die Daten weiterhin Daten ausgewählt aus einer Gruppe aufweisend Gerätedaten, Fehlercodes, Betriebsdaten, Umweltdaten, Verbrauchsmitteldaten, Netzwerkdaten, Behandlungsverlaufdaten aufweisen,

## Claims

1. A monitoring system (1) for at least one peritoneal dialysis appliance (PD1), whereby the monitoring system (1) receives from a peritoneal dialysis appliance (PD1) to be monitored via a first data communication network (NET1) traverse profile data, whereby the received data are stored with respect to a respective peritoneal dialysis appliance (PD1), whereby appliance specific at least individuals of the stored data are evaluated, whereby on basis of the evaluation an action selected from notifying a patient (P), notifying a medical specialized personnel (SP), notifying a quality officer (OB) is executed via a second data communication network (NET2), whereby the peritoneal dialysis appliance (PD1) to be monitored comprises a membrane pump, whereby vie evaluation of changes of the traverse profile data deterioration data of the membrane pump are determined.

2. The monitoring system (1) according to claim 1, **characterized in that** the first data communication network (NET1) is a wireless or wire-bound data communication network.

3. The monitoring system (1) according to claim 1 or 2, **characterized in that** the second data communication network (NET2) is a wireless or wire-bound data communication network.

4. The monitoring system (1) according to one of the preceding claims, **characterized in that** evaluation may be requested by a user.

5. The monitoring system (1) according to one of the preceding claims, **characterized in that** at least individuals of the stored data and evaluations of the respective stored data are retrievable via a Web-Browser.

6. Thee monitoring system (1) according to one of the preceding claims, **characterized in that** the data of the peritoneal dialysis appliance (PD1) is transferred encrypted.

7. The monitoring system (1) according to one of the preceding claims, **characterized in that** the notification is transmitted via a messaging service.

8. The monitoring system (1) according to one of the preceding claims, **characterized in that** via evaluation of changes in pressure profile(s) deterioration data are determined.

9. The monitoring system (1) according to one of the preceding claims, **characterized in that** the monitoring system (1) monitors a plurality of peritoneal dialysis appliance (PD1, PD2, ... PDN).

10. The monitoring system (1) according to one of the preceding claims, **characterized in that** the recording behavior of a peritoneal dialysis appliance (PD1) out of the plurality of peritoneal dialysis appliances (PD1, PD2, .... PDN) is adapted in response to a comparison of the actual failure rate to a reference failure rate.

11. The monitoring system (1) according to one of the preceding claims, **characterized in that** the data further comprises data selected from the group comprising appliance data, error codes, operating data, environmental data, consumable data, network data, treatment progress data.

## Revendications

1. Système de surveillance (1) pour au moins un appareil de dialyse péritonéale (PD1), dans lequel le système de surveillance (1) reçoit des données de profil d'itinéraire au moyen d'un premier réseau de transmission de données (NET1), dans lequel les données obtenues sont mémorisées en relation avec une machine de dialyse péritonéale (PD1) respective, dans lequel au moins certaines des données mémorisées sont évaluées en relation avec le dispositif, dans lequel une action sélectionnée sur la base de l'évaluation parmi la notification à un patient (P), notification à un médecin spécialiste (FP), notification au personnel de service (SP), notification à un responsable qualité (QB) au moyen d'un deuxième réseau de transmission de données (NET2) est exécutée, dans lequel l'appareil de dialyse péritonéale (PD1) à surveiller présente une pompe à membrane, dans lequel les données d'usure de la pompe à membrane sont déterminées en évaluant les modifications du profil d'itinéraire.

2. Système de surveillance (1) selon la revendication 1, **caractérisé en ce que** le premier réseau de transmission de données (NET1) est un réseau de transmission de données sans fil ou filaire.

3. Système de surveillance (1) selon la revendication 1 ou 2, **caractérisé en ce que** le deuxième réseau de transmission de données (NET2) est un réseau de transmission de données sans fil ou filaire.

4. Système de surveillance (1) selon une des revendications précédentes, **caractérisé en ce que** l'évaluation peut être demandée par un utilisateur.

5. Système de surveillance (1) selon une des revendications précédentes, **caractérisé en ce qu'**au moins une partie des données mémorisées et des évaluations des données mémorisées pertinentes peuvent être appelées à l'aide d'un navigateur Web.

6. Système de surveillance (1) selon une des revendications précédentes, **caractérisé en ce que** les données de l'appareil de dialyse péritonéale (PD1) sont transmises sous forme cryptée.

7. Système de surveillance (1) selon une des revendications précédentes, **caractérisé en ce que** les notifications sont envoyées à l'aide d'un service de messagerie.

8. Système de surveillance (1) selon une des revendications précédentes, dans lequel les données d'usure sont déterminées en évaluant les variations de profil(s) de pression.

9. Système de surveillance (1) selon une des revendications précédentes, **caractérisé en ce que** le système de surveillance (1) surveille un grand nombre d'appareils de dialyse péritonéale (PD1, PD2, ..., PDN).

10. Système de surveillance (1) selon une quelconque des revendications précédentes, **caractérisé en ce que** l'enregistrement du comportement d'une machine de dialyse péritonéale (PD1) parmi la pluralité de machines de dialyse péritonéale (PD1, PD2, ..., PDN) en réponse à une comparaison du taux d'erreurs actuel est ajustée avec un taux d'erreurs de référence.

11. Système de surveillance (1) selon une des revendications précédentes, **caractérisé en ce que** les données comprennent en outre des données sélectionnées dans un groupe comprenant des données de dispositif, des codes d'erreur, des données, données environnementales, données consommables, données de réseau, données d'historique de traitement.
